# EUROPEAN PATENT SPECIFICATION

(11) **EP 4 337 110 B1**
(45) Date of publication and mention of the grant of the patent: **02.04.2025**
(21) Application number: 22728416.3
(22) Date of filing: 09.05.2022
(51) Int. Cl.: A61B 17/12, A61M 60/126

(54) **IMPLANTABLE SHUNT REGULATION DEVICE AND SHUNT SYSTEM**
IMPLANTIERBARE SHUNT-REGELVORRICHTUNG UND SHUNT-SYSTEM
DISPOSITIF DE RÉGULATION DE DÉRIVATION IMPLANTABLE ET SYSTÈME DE DÉRIVATION

(30) Priority: 11.05.2021 EP 21173250
(43) Date of publication of application: 20.03.2024
(73) Proprietor: Universitätsklinikum Hamburg-Eppendorf, 20246 Hamburg (DE)
(72) Inventor: YILDIRIM, Yalin, 22529 Hamburg (DE); PECHA, Simon, 22529 Hamburg (DE)
(74) Representative: Stüven, Ralf
(86) International application number: PCT/EP2022/062426
(87) International publication number: WO 2022/238294

(56) References cited:
- US-A- 4 256 094
- US-A1- 2007 255 149

## Description

The invention relates to an implantable shunt regulation device and a shunt system.

Shunt systems for implantation in the human or animal body have been known for a long time and are used in many variants. These are usually tube systems with the help of which body fluid, for example blood or liquor, is conveyed from one region of the body, organ or part of an organ to another region of the body or another organ or part of an organ. Such shunt systems can, for example, have a drainage function in order to drain off otherwise accumulating fluid, for example cerebrospinal fluid in the case of hydrocephalus, or the function of diverting blood, for example from one blood vessel to another or from a heart ventricle into a blood vessel. Vascular shunt systems such as the Blalock-Taussig shunt and others are, for example, often used in adult and pediatric cardiac surgery (see, for example, Kiran U, Aggarwal S, Choudhary A, Uma B, Kapoor PM. The blalock and taussig shunt revisited. Ann Card Anaesth. 2017;20(3):323-330, doi:10.4103/aca.ACA_80_17; Yuan, Shi-Min, und Jing, Hua, Palliative procedures for congenital heart defects, 2009, Archives of Cardiovascular Diseases 102: 549-557, Doi:10.1016/j.acvd.2009.04.011; Jonas, Richard A., Indications and timing for the bidirectional Glenn shunt versus the fenestrated Fontan circulation, 1994, The Journal of Thoracic and Cardiovascular Surgery 108: 522-524, doi:10.1016/S0022-5223(94)70263-2; Alboliras ET, Chin AJ, Barber G, Helton JG, Pigott JD, Norwood WI. Pulmonary artery configuration after palliative operations for hypoplastic left heart syndrome. J Thorac Cardiovasc Surg. 1989 Jun;97(6):878-85. PMID: 2471019).

US 2007/0255149 A1 describes an apparatus for controlling blood flow through an aortopulmonary graft, wherein an implantable cuff is applied to the graft, the cuff including an expandable element that is selectively actuated to cause constriction of the graft during diastole.

US 4256094 A describes an arterial pressure control system for automatically adjusting arterial pressure to a predetermined level. The system includes an implantable inflatable cuff which is positioned and secured around an artery to be partially occluded. The inside surface of the cuff in contact with the artery wall is fitted with an inflatable annular liner. When a fluid such as water is injected into the cuff, the cuff inflates and compresses the vessel wall so as to partially occlude it.

Vascular shunt systems are also used in ventricular assist devices (VAD), where a shunt is inserted between a heart ventricle and an efferent blood vessel originating from the heart ventricle. VADs include a pump by means of which blood can be passed from the heart ventricle via the shunt into the efferent blood vessel to relieve the heart ventricle. Depending on the ventricle involved, such ventricular assist devices can be designed as a left ventricular assist device (LVAD), right ventricular assist device (RVAD) or biventricular assist device (BVAD). Left ventricular support devices are used, for example, in terminal heart failure as bridging therapy up to myocardial recovery or heart transplantation, and in certain cases also as long-term therapy (Kormos, R.L., Cowger, J, Pagani, F.D., Teuteberg, J.J., Goldstein, D.J., Jacobs, J.P., Higgins, R.S., Stevenson, L.W., Stehlik, J., Atluri, P., Grady, K.L., Kirklin, J.K., The Society of Thoracic Surgeons Intermacs database annual report: Evolving indications, outcomes, and scientific partnerships. J Heart Lung, Transplant, 2019. 38(2): p. 114-126, doi:10.1016/j.healun.2018.11.013; Mehra MR, Goldstein DJ, Uriel N, Cleveland JC Jr, Yuzefpolskaya M, Salerno C, Walsh MN, Milano CA, Patel CB, Ewald GA, Itoh A, Dean D, Krishnamoorthy A, Cotts WG, Tatooles AJ, Jorde UP, Bruckner BA, Estep JD, Jeevanandam V, Sayer G, Horstmanshof D, Long JW, Gulati S, Skipper ER, O'Connell JB, Heatley G, Sood P, Naka Y; MOMENTUM 3 Investigators, Two-Year Outcomes with a Magnetically Levitated Cardiac Pump in Heart Failure, N Engl J Med. 2018, 378(15):1386-1395. doi: 10.1056/NEJMoa1800866.). In ventricular assist devices, for example, in the event of pump malfunctions (e.g. pump thrombosis, electrical or mechanical malfunctions), a retrograde flow can occur via the vascular prosthesis (Kormos et al. 2019, above; Chrysant, George S.; Horstmanshof, Douglas A.; Snyder, Trevor; Chaffin, John S.; Elkins, C. Craig; Kanaly, Paul J.; Long, James W. Successful Percutaneous Management of Acute Left Ventricular Assist Device Stoppage, 2010, ASAIO J. 56: 483-485, doi: 10.1097/MAT.0b013e3181ed937f). Such a retrograde blood flow corresponds physiologically to a severe aortic valve insufficiency and further worsens the already poor hemodynamics in the emergency situation of a pump failure. In addition to a reduced ejection performance due to the pendulum flow and the associated cardiac function impairment, pulmonary edema can also occur and thus to an additional lung failure. So far, in rare cases, complex invasive catheter techniques have been used in such situations (see Knierim J, Heck R, Pieri M, Schoenrath F, Soltani S, Stawowy P, Dreysse S, Stein J, Müller M, Mulzer J, Dandel M, Falk V, Krabatsch T, Potapov E., Outcomes from a recovery protocol for patients with continuous-flow left ventricular assist devices, J Heart Lung Transplant. 2019, 38: 440-448, doi: 10.1016 / j.healun.2018.11.001). It would be desirable to have a shunt regulation system available with the aid of which such a retrograde flow can be more easily prevented or reduced.

In particular with a view to shunt surgery in children, it would also be desirable to have a shunt system available that can be adapted to the rapid growth of children more easily and without complex operations. Up to now, shunts have often had to be adjusted in order to conform them to the proportions of the growing children. This requires operations that are not only time-consuming, but also represent an additional burden for the children (see Yuan, Shi-Min, and Jing, Hua, Palliative procedures for congenital heart defects, 2009, Archives of Cardiovascular Diseases 102: 549-557 , Doi: 10.1016 / j.acvd.2009.04.011; Jonas, Richard A., Indications and timing for the bidirectional Glenn shunt versus the fenestrated Fontan circulation, 1994, The Journal of Thoracic and Cardiovascular Surgery 108: 522-524, doi: 10.1016 / S0022-5223 (94) 70263-2). Furthermore, at the moment, existing shunt systems can only be adapted to the intraoperative hemodynamic of the patient, which can differ from the postoperative situation. In operations like pulmonary banding, a postoperative adaption would be desirable to adapt the blood flow to the hemodynamic situation of the extubated patient.

The object of the present invention is to improve shunt systems, such that the above disadvantages can be avoided or reduced. In particular, it is the object of the invention to improve shunt systems in a manner that a retrograde flow can be prevented and invasive measures for adapting a shunt system to changing proportions, e.g., in children, can be avoided.

To solve the problem, the invention provides, in a first aspect, a shunt regulation device for implantation into a body, the device comprising
a) an electrically driven tube pinch valve,
b) electrical driving means for electrically driving the tube pinch valve
c) at least one first battery electrically connected to the electrical driving means for supplying the electrical driving means with electrical energy,
d) a first control unit electrically connected to the electrical driving means and configured to control the supply of electrical energy to the electrical driving means,
e) a first wireless receiving unit electrically connected to the first control unit and being configured to receive an external wireless signal, and
f) a closed first housing, part of the first housing comprising or forming at least part of a passage opening for receiving a flexible shunt tube or a body vessel,
wherein
- at least the electrical driving means for electrically driving the tube pinch valve is arranged in the closed first housing,
- the at least one battery (4) and the first control unit (5) are arranged, together with the electrical driving means (3), in the first housing (7), or are arranged together in a second housing,
- the tube pinch valve is arranged and configured in a manner that the pinch valve is able to pinch a flexible shunt tube or body vessel when arranged in the passage opening, and
- the tube pinch valve is adjustable from outside the body via the external wireless signal.

The term "shunt regulation device for implantation into a body" or "implantable shunt regulation device" relates to a device configured to regulate the flow of body fluid in an implantable shunt system, the device being suitable for implantation in a human or animal body.

The term "shunt system" relates to a tube system for transporting, e.g., conveying or redirecting, body fluid, which is suitable for implantation in a human or animal body. "Suitable for implantation in a human or animal body" means that at least the surfaces of the shunt system that come into contact with the body are biocompatible, i.e., have the property of exerting no or no significant negative influence on their metabolism in direct contact with living tissues. In addition, such materials preferably do not cause or promote any defense reactions in the body. Biocompatible materials are known to the person skilled in the art and include, for example, metals, metal alloys, ceramics and plastics. "Implantable" also means that components which are intended to carry electrical current are electrically isolated from the body.

The term "flexible shunt tube" as used herein is meant to relate in particular to a flexible tube made of a suitable biocompatible material, e.g., silicone, Dacron or Gore Tex, which can be compressed by external pressure on the tube wall, so that the cross-section of the tube and the flow rate of a fluid flowing through the tube is reduced. The tube is preferably compressible to such an extent that opposing inner surfaces of the tube wall can be brought together to such an extent that a complete closure takes place.

A "body" is understood here to mean a human or non-human animal body.

"Body fluid" is understood here to mean in particular extracellular body fluids such as blood, hemolymph, coelomic fluid, tissue fluid, lymph and cerebrospinal fluid.

The term "body vessel" relates to vessels in a body, for example blood vessels (arteries and veins) or lymph vessels.

The terms "pinch valve" or "tube pinch valve" relate to a valve using a pinching effect, i.e., the reduction of the cross-section of an elastic tubing or a body vessel by forcing opposite walls of the elastic tubing or vessel together, thus throttling or obstructing fluid flow, e.g., blood flow. A pinch valve may, for example, be configured to comprise opposing clamping jaws, which can be moved towards each other in a pinching motion, or as a wire loop or sling that can be pulled together.

The expression "adjustable from outside the body" in relation to the tube pinch valve of the shunt system according to the invention means that the contact pressure of the tube pinch valve and thus the flow cross-section of the shunt tube can be adjusted without surgical intervention in the body in which the shunt system is implanted. The terms "externally adjustable" or "externally controllable" may also be used synonymously. For example, the tube pinch valve can be adjusted from outside the body by means of electrical lines which extend outside the body, e.g., outside the thorax of a person, for example by means of an electronic control unit which is arranged outside the body and which is or can be connected to the electrical lines. The term "adjustable from outside the body via an external wireless signal" means that the tube pinch valve can be adjusted by means of a wireless connection from outside the body, e.g., via Bluetooth.

An "electrically driven tube pinch valve" is understood to mean a tube pinch valve that is directly or indirectly driven, i.e., can be opened and closed, by means of electrical energy. For this purpose, the tube pinch valve can be coupled to an electric motor, for example. "Directly driven" means that the tube pinch valve is opened and closed directly by an electrical driving means, e.g., an electric motor. "Indirectly driven" means that the tube pinch valve is opened and closed by an electric driving means, e.g., an electric motor, via a mechanical transmission.

The term "electrical driving means" relates to means converting electrical energy into mechanical energy. The term may, for example, relate to an electric motor. The term "electrical driving means" does not exclude any mechanical components, for example a transmission or transmission elements like gears, useful or necessary for converting the electrical energy into the desired mechanical energy, e.g., the pinching motion of a pinch valve.

A "battery" as used herein relates to any storage device for electrical energy and is understood to mean a device in which energy, for example electrical, electrochemical or chemical energy, is stored for the subsequent delivery of electrical energy. It can be a non-rechargeable or rechargeable storage device, for example a non-rechargeable battery, an accumulator or a capacitor. The terms "battery" or "battery pack" as used herein include rechargeable storage devices (e.g., accumulators). The term "battery pack" denotes an arrangement of several batteries and/or accumulators connected in a suitable manner in order to achieve a desired storage capacity. The use of the singular "battery" or "accumulator" is to be understood to include the use of several batteries and/or accumulators. Conversely, the use of the plural "batteries" or "accumulators" should not be understood to mean that the use of only one battery or accumulator should be excluded.

The term "portable", if used herein in relation to a battery or a control unit, means that the storage device or the control unit can be worn on the body by a person in whom a shunt system according to the invention is implanted, i.e., is designed so that it can easily be carried around even by a person weakened due to an illness, for example heart disease, for example in a holder attached to the body.

The term "closed housing" as used herein relates to a housing that is essentially closed off from the body environment in which it is implanted. "Closed off from the body environment" includes that body fluid, cells or tissue are prevented from entering the interior of the housing. This does, however, not exclude a housing having a passage opening through which a tube of a suitable diameter can be passed.

A "control unit" is understood to mean an electronic module that is used to control components of the shunt system or the shunt regulation device, for example the tube pinch valve and/or other shunt system components, for example a pump.

The terms "wireless receiving unit" or "wireless transmitting unit" relates to an electronic component being configured to receive or transmit wireless signals, e.g., radio signals like Bluetooth signals. This does not exclude that a wireless receiving unit may be configured to also being able to transmit a wireless signal, or that a wireless transmitting unit may be configured to also being able to receive a wireless signal.

A "ventricular assist device" (VAD) is understood here to mean a shunt system that uses a pump to transport blood from a heart ventricle into an efferent blood vessel connected to the heart ventricle. With a left ventricular support system (LVAD), blood is pumped from the left ventricle through an inlet cannula (also "inflow graft") into an outlet cannula (also "outflow graft"), which is inserted into the aorta, usually the ascending part of it, i.e., the aorta ascendens, whereby left ventricular blood can be transported into the aorta bypassing the aortic valve (valva aortae). The pump takes over at least part of the pumping capacity of the left ventricle and thus relieves it. With a right ventricular support system (RVAD), blood is pumped from the right ventricle bypassing the pulmonary valve (Valva trunci pulmonalis) into the pulmonary artery (arteria pulmonalis). A biventricular assist system (BVAD) is a combination of an LVAD and an RVAD.

The implantable shunt regulation device according to the invention enables the flow of body fluids, for example blood, to be adjusted externally and can be used as part of shunt systems like artificial heart systems or ventricular assist devices. It can also be used to regulate the flow of body fluid, e.g., blood or lymph, in natural body vessels. The implantable shunt regulation device according to the invention enables, for example, the prevention or at least reduction of a retrograde flow in ventricular assist devices, in particular left ventricular assist devices, in that a shunt tube used in these devices is clamped from outside the body by closing the tube pinch valve and thus the unphysiological blood return flow from the aorta in the direction of the left ventricle is prevented. The invention provides a simple and reliable means of controlling the flows of body fluids that are drained or redirected by means of a shunt system. The adjustment of the tube pinch valve, for example its partial or complete closure, can be carried out without surgical intervention in the body.

Essential components for the operation of the shunt regulation device according to the invention, e.g., the electrical driving means for electrically driving the tube pinch valve, the energy source and electronic components for the operation and control of the tube pinch valve, are encapsulated in one or more housings. The shunt regulation device of the invention does not need any wiring that would have to be led out of the body, but can be remotely operated and controlled via a wireless connection, e.g., a Bluetooth connection. The shunt regulation device is thus configured as a self-contained unit. **In** the shunt regulation device according to the invention, adhesions by foreign body reactions are essentially avoided, since they could otherwise interfere with the mechanical operation of the tube pinch valve. The housing(s) and the tube pinch valve are so designed that body fluid or tissue is prevented from entering the interior of the housing(s). A housing comprises or forms a passage opening for a shunt tube or a body vessel, allowing a shunt tube or body vessel to be positioned in relation to the tube pinch valve in a manner that the shunt tube or body vessel can be clamped, e.g., between clamping jaws of the tube pinch valve.

When used in a left ventricular assist system, for example, closing the tube pinch valve of the implantable shunt regulation device according to the invention can help to immediately stabilize the hemodynamics of the patient by reducing the pendulum volume or blood reflux and to prevent pulmonary edema. The initial stabilization of the hemodynamics can then allow further therapy measures, for example extracorporeal membrane oxygenation (ECMO), or a change of the pump in the left ventricular assist device. The implantable shunt regulation device according to the invention can also be used for evaluating an LVAD explantation after the myocardial function has recovered. Here, by simulating a pump stop with clamping of a shunt system functioning as an outflow graft, a situation without LVAD can be simulated and the hemodynamic function can be evaluated. For this purpose, invasive catheter techniques were used in the past, which are no longer required due to the invention.

In addition, the implantable shunt regulation device according to the invention can be used in patients who no longer need the LVAD. In cases in which LVAD deactivation is sought without explanting the LVAD, the implantable shunt regulation device according to the invention can be used to permanently close the outflow graft to prevent the backflow of blood from the aorta via the outflow graft. Today there is no standardized solution for this. However, individual cases have been described in which the outflow graft was closed by complex catheter-interventional procedures (Zeigler SM, Sheikh AY, Lee PH, Desai J, Banerjee D, Oyer P, et al. A novel, catheter-based approach to left ventricular assist device deactivation after myocardial recovery, Ann Thorac Surg. 2014; 98: 710-3). This can be solved much more easily with the shunt regulation device according to the invention and a complex catheter-supported occlusion of the graft is no longer necessary. In addition, patients can be spared an operation.

The implantable shunt regulation device according to the invention can also be used advantageously in the field of pediatric cardiac surgery, and replace or supplement previous shunt systems (e.g., Blalock-Taussig shunt, Sano shunt, Glenn shunt, Mee shunt), in which flow adjustment has not been possible up to now. The adaptation of the shunt sizes, for example required due to the rapid growth of children, which has hitherto been carried out surgically by replacing a larger shunt, can be carried out with the aid of the shunt regulation device according to the invention without additional invasive intervention. For example, initially larger shunts could be used, which can then be continuously adjusted from outside the body as required and can deliver a demand-oriented flow. The number of revision operations to adapt the shunt sizes can thus be significantly reduced. Another advantage is the regulation of the shunt flow in a postoperative setting. Currently, the shunt sizes and hemodynamic parameters can only be determined intraoperatively under anesthesia. Often these parameters are affected by the anesthesia. The invention here offers a possibility of readjusting the shunt flow under physiological conditions. The shunt regulation device of the invention can also directly be used for controlling the flow of body fluid, e.g., blood or lymph, through body vessels. In such a case, the use of a shunt tube is not necessary.

In a preferred embodiment of the shunt regulation device of the invention, at least part of the tube pinch valve, the electrical driving means for electrically driving the pinch valve, including necessary transmission components, the at least one battery and the first control unit are arranged together in the first housing. In this embodiment, all components necessary for operating the pinch valve are arranged in a single closed housing. Preferably, the whole tube pinch valve, the electrical driving means for electrically driving the pinch valve, including necessary transmission components, the at least one battery and the first control unit are arranged together in the first housing. The expression, according to which the whole tube pinch valve is arranged, together with the other components, in the first housing does not exclude that parts of the pinch valve, for example, a clamping jaw, are temporarily, e.g., during a pinching motion, located outside the housing, e.g., within the passage opening.

Alternatively, the whole tube pinch valve or at least part of it and the electrical driving means for electrically driving the pinch valve are arranged in a first housing, and the at least one battery and the first control unit are arranged together in a second closed housing. In this embodiment, the components for the electrical supply and control of the tube pinch valve are separated from the tube pinch valve and the associated electrical driving means, and arranged in a separate (second) closed housing. This allows for more flexibility and better miniaturization of the shunt regulation device of the invention. Further, the separate housing including the components for energy supply, can be separately replaced, if necessary, without the need for removal of the first housing with its components. Of course, the first and second housing are electrically connected to each other, e.g., via a cable, such that the electrical driving means arranged in the first housing can be supplied with electrical energy from the battery or batteries arranged in the second closed housing.

The passage opening for receiving a shunt tube can be arranged in a base part of the first housing, or the passage opening can be formed by an extension of the housing, for example, by wall parts extending from a side wall of a base part of the housing. Wall parts of the housing may, for example, extend from a base part of the housing such that, in cross-section, a generally u-shaped or arc-shaped structure results forming the walls of a passage opening. In this embodiment, the shunt valve may be configured as a wire sling that is arranged in or around the passage opening such that a shunt tube or body vessel can be passed through the sling. The wire sling can be arranged in corresponding ducts arranged in the housing extension forming the passage opening. In this embodiment, the electrical driving means are preferably configured in a manner that the ends of the sling can be rolled up in order to tighten the sling. The housing extension may be configured to be reversibly detachable from the housing, at least on one side. In this embodiment, preferably at least one end of the sling can also be reversibly detached from the electrical driving means. This allows for arrangement of the shunt regulation device of the invention without the need for operationally cutting a vessel. Embodiments of the shunt regulation device of the invention having the passage opening formed by a housing extension are particularly useful for directly regulating fluid flow through body vessels, e.g., the pulmonary artery.

In a further aspect, the invention also relates to a shunt system comprising an implantable shunt regulation device according to the invention and a second control unit for arrangement outside the body, the second control unit being electrically connected to a first wireless transmitting unit configured to transmit a wireless signal to the first wireless receiving unit from outside the body for remotely adjusting the tube pinch valve. The second control unit and the first wireless transmitting unit can, for example, be arranged in a remote control. The first wireless receiving unit and the first wireless transmitting unit are adapted to each other in a suitable manner in order to enable wireless data transmission.

The tube pinch valve can be designed to be continuously adjustable, and the first and second control unit can be configured so that the tube pinch valve can be remotely closed or opened in a precisely defined manner via a wireless connection. The first control unit is directly or indirectly electrically connected to the electrical driving means in order to allow it to control the operation of the electrical driving means and thus the tube pinch valve. Via the first control unit the tube pinch valve can also report data back to the second control unit via the wireless connection, for example about its exact opening or closing state. Any malfunction of the tube pinch valve can also be transmitted to the control unit and, if necessary, trigger a warning message, for example.

In a preferred embodiment of the shunt system of the invention, the shunt system further comprises an implantable flexible shunt tube for transporting body fluid, the shunt tube having a first open end and a second open end. The flexible shunt tube is preferably arranged in the passage opening of the housing such that the tube pinch valve is arranged between the first end and the second end of the shunt tube.

In a particularly preferred embodiment, the shunt system according to the invention comprises an implantable pump, which is connected on its inlet side to an inlet cannula for implantation in a heart ventricle and on its outlet side to the first open end of the flexible shunt tube, the second open end of the flexible shunt tube being implantable into blood vessel extending from the heart ventricle.

In this embodiment, the shunt system according to the invention is configured as a ventricular assist device (VAD). This embodiment can function and be used as a left ventricular assist device (LVAD), right ventricular assist device (RVAD) or as a bilateral ventricular assist device (BVAD). Use as a left ventricular assist device is preferred. The combination of shunt tube and tube pinch valve described above replaces the outlet catheter (outflow graft) known from the prior art ventricular assist devices, which are not controllable with regard to their flow rate, and enables targeted control, e.g., throttling, the flow of the blood pumped by the pump into the connected blood vessel, e.g., the aorta. In pediatric cardiac surgery, the shunt system of the invention configured as a ventricular assist device enables an adaptation to the changing size relationships, for example by initially using an outlet catheter that is comparatively too large at the time of transplantation and throttling its flow using the tube pinch valve, and later increasing the flow cross-section of the shunt tube by gradually opening the tube pinch valve with increasing growth and the accompanying enlargement of the physical structures of the affected child, thus increasing the blood flow into the connected blood vessel, e.g. the aorta.

The implantable pump can be configured, for example, as an axial pump or centrifugal pump, as known from the prior art (see, for example, Moazami N, Fukamachi K, Kobayashi M, Smedira NG, Hoercher KJ, Massiello A, Lee S, Horvath DJ, Starling RC. Axial and centrifugal continuous-flow rotary pumps: a translation from pump mechanics to clinical practice. J Heart Lung Transplant. 2013, 32(1):1-11. doi: 10.1016/j.healun.2012.10.001).

In the embodiment of the shunt system according to the invention embodied as a VAD, it is preferred that the pump can be supplied with electrical energy from outside the body via a connecting cable, preferably via a portable battery. Preferably, the pump is also electrically connected to a pump control unit arranged outside the body, the pump control unit being preferably also portable.

In this embodiment of the shunt system according to the invention, it is further preferred that the pump is electrically connected to the pump control unit via the connecting cable connecting the pump with the battery. In this way, for example, the pump can also be controlled, or data on the status of the pump can be transmitted to the control unit. The pump control unit may be configured to be itself remotely controlled, e.g., via a wireless connection like a radio or infrared connection.

The implantable shunt regulation device and/or the shunt system according to the invention can advantageously be used in methods for the therapeutic treatment of a human or non-human animal, for example in the case of cardiac insufficiency due to dysfunction of an LVAD or corrective shunt surgery in pediatric patients.

The invention is explained in more detail below, for illustrative purposes only, with reference to the attached figures.
Fig. 1 Simplified representation of an embodiment of a shunt system according to the invention.
Fig. 2 View into the interior of an embodiment of a shunt regulation device according to the invention in a first ("open") state.
Fig. 3. View into the interior of an embodiment of a shunt regulation device according to the invention in a second ("closed") state.
Fig 4-6. Simplified views of a further embodiment of a shunt regulation device according to the invention with a housing extension forming a passage opening.

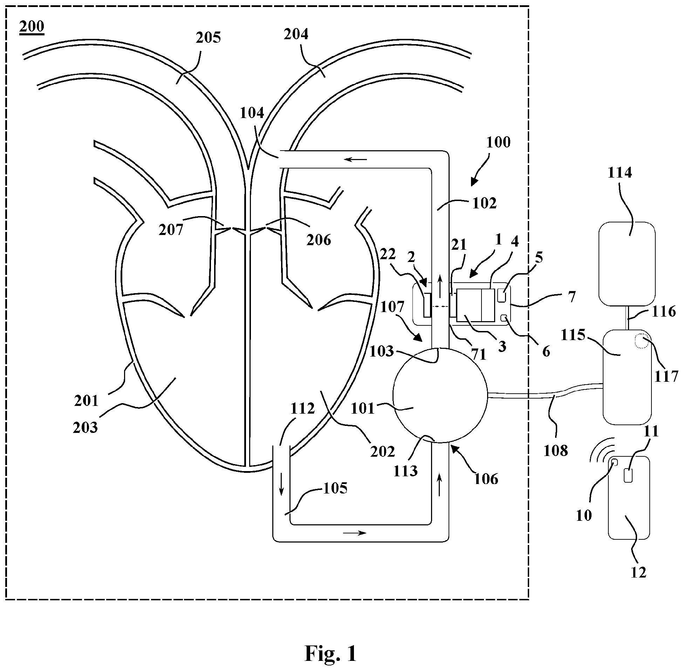

Figure 1 shows a simplified and schematic representation of an embodiment of a shunt system 100 according to the invention. In the embodiment shown here, the shunt system 100 according to the invention is configured as a left ventricular assist device system (LVAD) and is implanted in a body 200. The body 200 is simplified as a rectangle and the body surface is symbolized by dashed lines. In addition to a shunt regulation device 1 according to the invention comprising an electrically driven tube pinch valve 2, the LVAD comprises a flexible shunt tube 102 and a pump 101, here of the centrifugal pump type. On its inlet side 106, the pump 101 is connected to the second open end 113 of an inlet cannula 105. With its open first end 112, the inlet cannula 105 is implanted in the tip of the left ventricle 202 of a heart 201, which is shown here very schematically. An "open second end" 113 here means that the cannula 105 is open to the pump 101, but not to the rest of the environment, i.e., towards the body 200. The inlet side 106 of the pump 101 is thus in fluid communication, via the second open end 113 of the inlet cannula 105, with the first open end 112 of the inlet cannula 105 implanted in the left ventricle 202. Arrows here indicate the direction of flow of the pumped blood. The shunt tube 102 ends with its second open end 104 in the aorta 204, specifically, in the natural flow direction of the blood behind the aortic valve 206, so that the aortic valve 206 is bypassed. The shunt tube 102 is connected to the outlet side 107 of the pump 101 by its first open end 103. Here, too, "open end" simply means that the shunt tube 102 is open to the pump 101 and not to the body 200. With the aid of the shunt system 100 according to the invention, blood can be drawn from the left ventricle 202 and pumped via the pump 101 into the Aorta 204 for relieving the ventricle 202. The shunt system 100 according to the invention could accordingly also be designed to support the right ventricle 203 and would then pump blood from the right ventricle 203 into the pulmonary artery 205, bypassing the pulmonary valve 207.

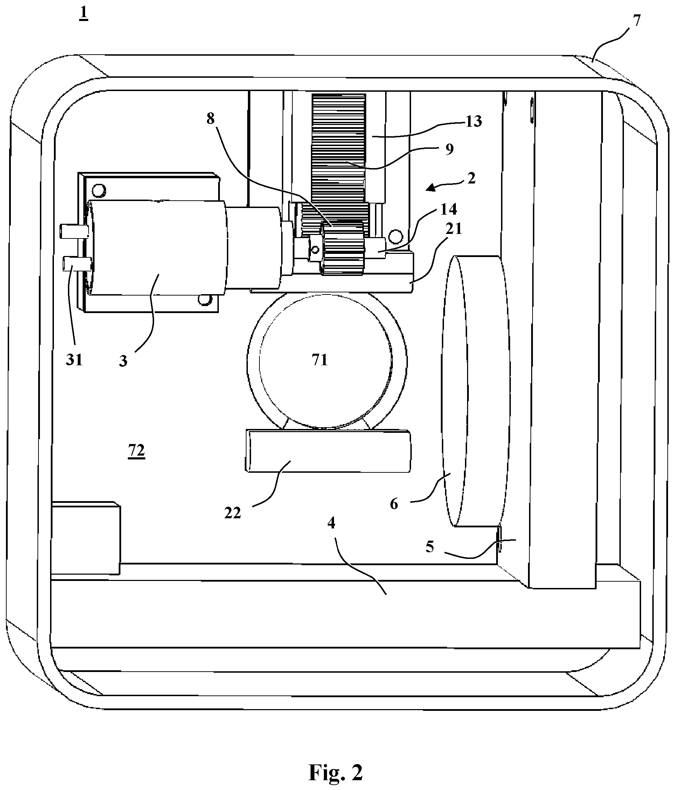

The shunt regulation device 1 according to the invention here comprises a tube pinch valve 2 connected to electrical driving means 3, being or comprising e.g., an electric motor, and two clamping jaws 21, 22 that can be brought together and apart in order to pinch a flexible shunt tube 102 arranged between the clamping jaws 21, 22. The tube pinch valve 2 is shown here in the fully open state (see also Fig. 2). The clamping jaws 21, 22 can be brought together, as a result of which the opposing walls of the shunt tube 102 located between the clamping jaws 21, 22 move towards one another and the shunt tube 102 can be completely or partially closed, i.e., can be completely or partially pinched off. When the phrase is used here that the "clamping jaws 21, 22 can be brought together", this includes that only one of the clamping jaws 21, 22 is moved in relation to the other clamping jaw 22, 21. The electrical driving means 3, i.e., the electric motor in this embodiment, is electrically connected to a first battery 4, the battery 4 supplying the electrical driving means 3 with electric energy for moving the clamping jaws 21, 22 of the pinch valve 2. The electric motor is mechanically coupled to the pinch valve 2 (see Fig. 2) via a transmission.

The shunt regulation device 1 of the invention further comprises a first control unit 5 and a first wireless receiving unit 6. The first control unit 5 is electrically connected to the electrical driving means 3 and is configured to control the supply of electrical energy to the electrical driving means 3. The first control unit 5 is electrically connected to a first wireless receiving unit 6 being configured to receive an external wireless signal, e.g., a radio signal such as a Bluetooth signal. Via the wireless signal the tube pinch valve 2 can be controlled from outside the body 200, i.e., closed, opened or be set to a specific opening state in order to regulate a flow of body fluid through a shunt tube 102 implanted in the body 200. The pinch valve 2, the electrical driving means 3 for electrically driving the tube pinch valve 2, the battery 4 for supplying the electrical driving means 3 with electrical energy, the first control unit 5 and the first wireless receiving unit 6 are here all arranged in a closed first housing 7, the closed first housing 7 having a passage opening 71 through which a flexible shunt tube 102 can be passed. It is, however, also possible to arrange the tube pinch valve 2, or at least part of it, and the electrical driving means 3 for electrically driving the tube pinch valve 2 in the closed first housing 7, and the battery 4, the first control unit 5 and the first wireless receiving unit 6 in a separate closed second housing, or to arrange the tube pinch valve 2, or at least part of it, the electrical driving means 3 for electrically driving the tube pinch valve 2, the first control unit 5 and the first wireless receiving unit 6 in within the closed firs housing 7, and only the battery 4 within a closed second housing. The shunt regulation device 1 is thus a self-sustaining unit that can be implanted into a body 200 without the need for any cabling extending from the shunt regulation device 1 to the outside of the body 200. The shunt regulation device 1 can be fully operated, i.e., the tube pinch valve 2 be (partially) closed or (partially) opened, from outside the body 200 via a wireless connection. It may only be necessary to replace the battery 4 from time to time.

The first wireless receiving unit 6 is configured to receive a wireless signal from a first wireless transmitting unit 10 arranged outside the body 200, e.g., within a remote control 12. The wireless transmitting unit 10 is electrically connected to a second control unit 11 also arranged within the remote control 12 and being configured to control the first wireless transmitting unit 10. The wireless connection, e.g., Bluetooth connection, between the first wireless receiving unit 6 and the first wireless transmitting unit 10 serves to remotely control the shunt regulation device 1, especially to control opening and closing the tube pinch valve 2. The wireless connection can also be used in both directions, i.e., also for the transmission of data from the shunt regulation system 1, e.g., data relating to the opening state of the tube pinch valve 2 or the status of the battery 4. The first wireless receiving unit 6 may thus be configured as a first wireless receiving/transmitting unit 6 that is also able to transmit a wireless signal to the first wireless transmitting unit 10, and the first wireless transmitting unit 10 may also be configured as a receiving/transmitting unit, i.e., a unit being able to both transmit and receive a wireless signal, e.g., a Bluetooth signal.

In the embodiment of a shunt system 100 according to the invention shown in Figure 1 the pump 101 is connected via a first connecting cable 108 to a pump control unit 115 arranged outside the body 200 and to a battery 114, e.g., a battery pack, arranged outside the body 200. The pump control unit 115 and the battery 114 are interconnected here via a connecting cable 116. The pump control unit 115 includes a second wireless receiving unit 117, which may be configured to receive a wireless signal from the first wireless transmitting unit 10 or from a second wireless transmitting unit (not shown). The pump control unit 115 controlling the pump 101 can thus be controlled via a wireless connection, e.g., a radio or infrared connection. The pump 101 receives control signals and electrical energy for its operation via the connecting cable 108. The connecting cable 108 can also be used to transmit data from the pump, e.g., the pump rate, to the pump control unit 115.

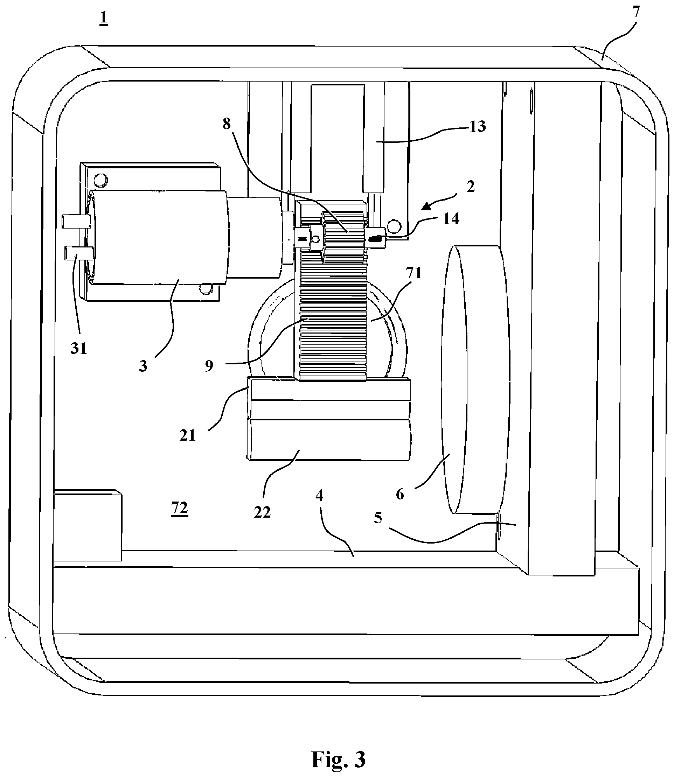

Figure 2 and 3 show an embodiment of a shunt regulation device 1 of the invention. In this embodiment, an electrically driven tube pinch valve 2, electrical driving means 3 (here an electric motor) for electrically driving the tube pinch valve 2, a first battery 4, a first control unit 5 and a first wireless receiving unit 6 are arranged together in a common first housing 7. Shown is a view into the interior of the first housing 7 with the cover (not shown) removed and comprising part of the components of the shunt regulation device 1. No wiring, e.g., for the supply of the electrical driving means 3 is shown. Only the pins 31 for connecting the wiring from the battery 4 to the electrical driving means 3, i.e., the electric motor, are shown. Within the first housing 7, the first control unit 5 and the first wireless receiving unit 6 are arranged. The pinch valve 2 comprises first and second clamping jaws 21, 22, the second clamping jaw 22 being fixedly attached to the inner wall of the first housing 7 below a passage opening 71 arranged in a base part 72 of the first housing 7, through which a shunt tube 102 can be passed such that the shunt tube 102 is placed between the clamping jaws 21, 22. In this embodiment, the housing 7 does not have a housing extension. The first clamping jaw 21, i.e., the clamping jaw 21 opposing the fixed second clamping jaw 22, is attached to a gear rack 9 running in guide rails 13. The gear rack 9 and the first clamping jaw 21 attached thereto can be moved in the direction of the second clamping jaw 22 by means of a gear wheel 8 mounted on a rotatable shaft of the electrical driving means 3 and engaged with the gear rack 9. The first clamping jaw 21 can be moved towards the second clamping jaw 22 via the combination of gear wheel 8 and gear rack 9 by means of the electrical driving means 3. The cover (not shown) has a corresponding passage opening for passing a shunt tube 2 through. In figure 1, the tube pinch valve 2 is shown in an open position, i.e., the clamping jaws 21, 22 of the pinch valve 2 are spaced apart from each other in order to enable the passing through of a shunt tube 102. In figure 3, the tube pinch valve 2 is shown in a closed position, i.e., a state, in which the clamping jaws 21, 22 are in contact with each other. The embodiment described is particularly useful for adult patients, although it can also be used for pediatric patients. It is preferably be used in connection with a shunt tube 102, but can also be used to directly regulate the flow of a body vessel, e.g., a blood vessel.

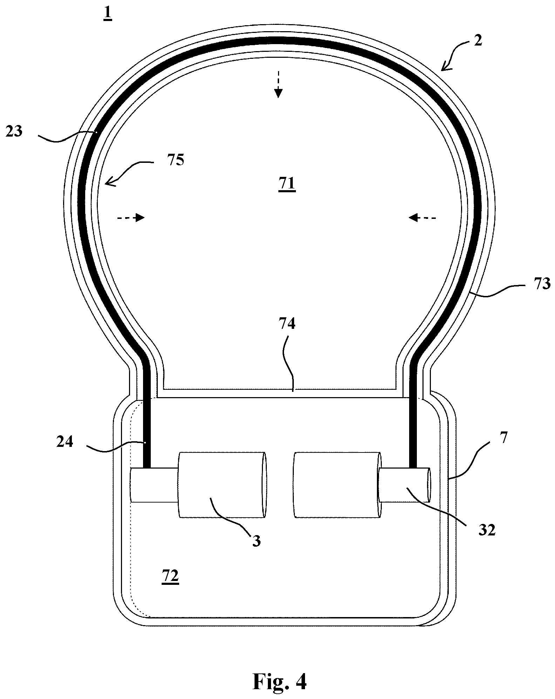

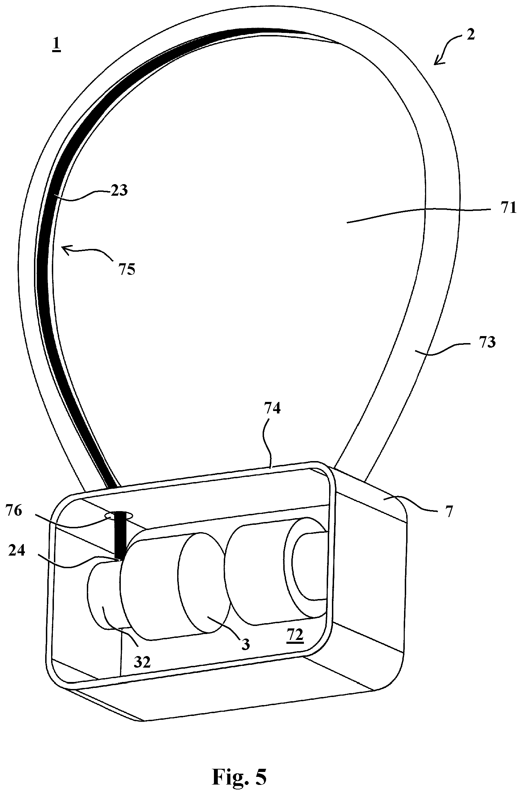

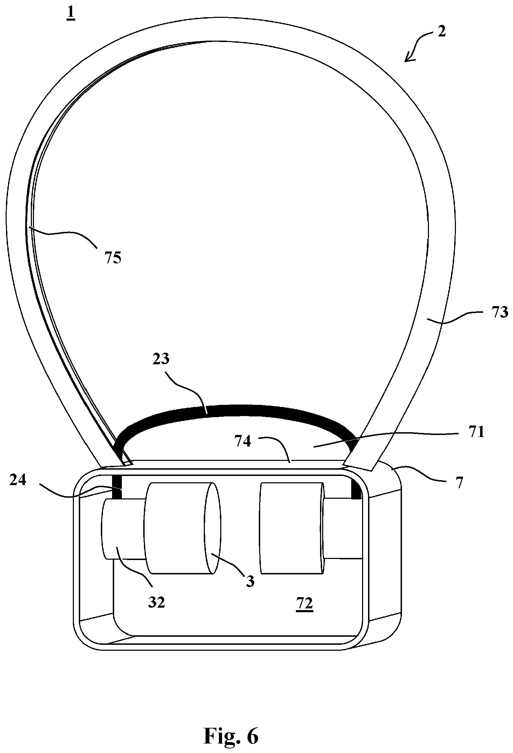

Figures 4 to 6 show, only schematic and simplified, different views of another embodiment of a shunt regulation device 1 of the invention. In this embodiment, the electrically driven tube pinch valve 2 and electrical driving means 3 (here two electric motors) are arranged in the first housing 7, whereas the first battery 4, the first control unit 5 and the first wireless receiving unit 6 are together arranged in a second housing (not shown). The pinch valve 2 is configured as a wire sling 23, at least part of which is arranged in a generally u-shaped or arc-shaped extension 73 of the first housing 7, extending from a side wall 74 of a base part 72 of the first housing 7 comprising the electrical driving means 3 and the ends 24 of the wire sling 23. The ends 24 of the wire sling 23 are passed through openings 76 (see Fig. 5) in the base part 72 of the first housing 7 and are attached to the shafts 32 of the two electric motors, such that the wire sling 23 can be tightened by wrapping the ends 24 around or within the shafts 32 of the electric motors. The housing extension 73 is open in the direction of the passage opening 71, thus forming a groove or channel, such that the wire sling 23 placed therein in the open position of the tube pinch valve 2 can protrude from the housing extension 73 through the slot-shaped opening 75 when tightened (as indicated by the dashed arrows in Fig. 4), as can better be seen in Figure 6, where the wire sling 23 is shown in a closed position. A flexible shunt tube 102 or a body vessel (not shown in the figure), e.g., a blood vessel, can be passed through the passage opening 71 and the cross-section of the shunt tube 102 or the vessel can be reduced (or enlarged) in order to reduce (or enlarge) the fluid flow through the tube 102 or vessel by tightening or loosening the wire sling 23. This embodiment is particularly useful for use in pediatric patients, and for directly regulating the fluid flow of a body vessel, although it is by no means restricted to this use. It can also advantageously be used for adult patients and/or together with a shunt tube 102. The housing extension 73 can be configured to be detachable from the base part 72 of the first housing 7, such that it can be implanted without the need for cutting the vessel, the fluid flow of which is to be regulated by the device. Further, the housing extension 73 can also be an optionally flexible tube having an opening in the direction of the passage opening 71.

In a test with an artificial circulatory model, which was carried out with two shunt systems 1 according to the invention, which were operated as LVADs, it was shown that the shunt regulation device 1 according to the invention can efficiently prevent backflow via the outflow prosthesis (outflow graft). In comparison to an experimental setup with LVADs according to the prior art, it was possible to show that the reflux was reduced from 1.9 1/min ± 0.4 l/min to 0 l/min. Furthermore, it could be shown that the mean systemic aortic pressure in the artificial circulatory model after LVAD failure with an LVAD designed according to the invention was significantly higher (69 mmHg vs. 54 mmHg). The ability of a stepless flow regulation by a shunt regulation device 1 according to the invention was also confirmed.

## Claims

1. A shunt regulation device (1) for implantation into a body (200), comprising
a) an electrically driven tube pinch valve (2),
b) electrical driving means (3) for electrically driving the tube pinch valve (2)
c) at least one first battery (4) electrically connected to the electrical driving means (3) for supplying the electrical driving means (3) with electrical energy,
d) a first control unit (5) electrically connected to the electrical driving means (3) and configured to control the supply of electrical energy to the electrical driving means (3),
e) a first wireless receiving unit (6) electrically connected to the first control unit (5) and being configured to receive an external wireless signal, and
f) a closed first housing (7), part of the first housing (7) comprising or forming at least part of a passage opening (71) for receiving a flexible shunt tube (102) or a body vessel,
wherein
- at least the electrical driving means (3) for electrically driving the tube pinch valve (2) is arranged in the closed first housing (7),
- the at least one battery (4) and the first control unit (5) are arranged, together with the electrical driving means (3), in the first housing (7), or are arranged together in a second housing,
- the tube pinch valve (2) is configured in manner that the pinch valve (2) is able to pinch a flexible shunt tube (102) or body vessel when arranged in the passage opening (71), and
- the tube pinch valve (2) is adjustable from outside the body (200) via the external wireless signal.

2. A shunt regulation device (1) according to claim 1, wherein at least part of the tube pinch valve (2), the electrical driving means (3), the at least one battery (4) and the first control unit (5) are arranged together in the first housing (7).

3. A shunt regulation device (1) according to claim 1 or 2, wherein a base part (72) of the housing (7) comprises the passage opening (71).

4. A shunt regulation device (1) according to claim 1 or 2, wherein the passage opening (71) is formed by an extension (73) of the housing (7), extending from a side wall (74) of the first housing (7).

5. A shunt regulation device (1) according to claim 4, wherein the tube pinch valve (2) comprises a wire sling (23) being arranged in or around the passage opening (71).

6. The shunt regulation device (1) according to one of the preceding claims, wherein the electrical driving means (3) is an electric motor.

7. A shunt system (100), comprising a shunt regulation device (1) according to claim 1 and a second control unit (11) for arrangement outside the body (200), the second control unit (11) being electrically connected to a first wireless transmitting unit (10) configured to transmit a wireless signal to the first wireless receiving unit (6) from outside the body (200) for remotely adjusting the tube pinch valve (2).

8. The shunt system (100) according to claim 7, further comprising an implantable flexible shunt tube (102) for transporting body fluid, with a first open end (103) and a second open end (104).

9. The shunt system (100) according to claim 8, wherein the flexible shunt tube (102) is arranged in the passage opening (71) of the housing (7) such that the tube pinch valve (2) is arranged between the first end (103) and the second end (104) of the shunt tube (102).

10. The shunt system (100) according to one of claims 7 or 9, wherein the second control unit (11) and the first wireless transmitting unit (10) are arranged in a remote control (12).

11. The shunt system (100) according to one of claims 7 to 10, further comprising an implantable pump (101), which on its inlet side (106) is connected to an inlet cannula (105) for implantation in a heart ventricle (202, 203) and on its outlet side (107) is connected to the first open end (103) of the flexible shunt tube (102), the second open end (104) of the shunt tube (102) being implantable in a blood vessel (204, 205) originating from the heart ventricle (202, 203).

12. The implantable shunt system (100) according to claim 11, wherein the pump (101) can be supplied with electrical energy from outside the body (200) via a connecting cable (108), preferably via a second battery (109).

13. The implantable shunt system (100) according to claim 12, wherein the pump (4) is electrically connected to a pump control unit (115) arranged outside the body (200) via the connecting cable (108).

## Patentansprüche

1. Shunt-Regulierungseinrichtung (1) zur Implantation in einen Körper (200), die Folgendes umfasst:
a) ein elektrisch angetriebenes Schlauchquetschventil (2),
b) elektrische Antriebsmittel (3) zum elektrischen Antreiben des Schlauchquetschventils (2),
c) mindestens eine erste Batterie (4), die elektrisch mit den elektrischen Antriebsmitteln (3) verbunden ist, um die elektrischen Antriebsmittel (3) mit Elektroenergie zu versorgen,
d) eine erste Steuereinheit (5), die elektrisch mit den elektrischen Antriebsmitteln (3) verbunden und dafür konfiguriert ist, die Zufuhr von Elektroenergie zu den elektrischen Antriebsmitteln (3) zu steuern,
e) eine erste Drahtlos-Empfangseinheit (6), die elektrisch mit der ersten Steuereinheit (5) verbunden ist und dafür konfiguriert ist, ein externes Drahtlos-Signal zu empfangen, und
f) ein geschlossenes erstes Gehäuse (7), wobei ein Teil des ersten Gehäuses (7) zumindest einen Teil einer Durchgangsöffnung (71) zum Aufnehmen eines flexiblen Shunt-Schlauchs (102) oder eines Körpergefäßes umfasst oder bildet,
wobei
- zumindest die elektrischen Antriebsmittel (3) zum elektrischen Antreiben des Schlauchquetschventils (2) in dem geschlossenen ersten Gehäuse (7) angeordnet sind,
- die mindestens eine Batterie (4) und die erste Steuereinheit (5), zusammen mit den elektrischen Antriebsmitteln (3), in dem ersten Gehäuse (7) angeordnet sind oder zusammen in einem zweiten Gehäuse angeordnet sind,
- das Schlauchquetschventil (2) auf eine solche Weise konfiguriert ist, dass das Schlauchquetschventil (2) dazu in der Lage ist, einen flexiblen Shunt-Schlauch (102) oder ein Körpergefäß abzuklemmen, wenn sie in der Durchgangsöffnung (71) angeordnet sind, und
- das Schlauchquetschventil (2) über das externe Drahtlos-Signal von außerhalb des Körpers (200) einstellbar ist.

2. Shunt-Regulierungseinrichtung (1) nach Anspruch 1, wobei zumindest ein Teil von dem Schlauchquetschventil (2), den elektrischen Antriebsmitteln (3), der mindestens einen Batterie (4) und der ersten Steuereinheit (5) zusammen in dem ersten Gehäuse (7) angeordnet sind.

3. Shunt-Regulierungseinrichtung (1) nach Anspruch 1 oder 2, wobei ein Basisteil (72) des Gehäuses (7) die Durchgangsöffnung (71) umfasst.

4. Shunt-Regulierungseinrichtung (1) nach Anspruch 1 oder 2, wobei die Durchgangsöffnung (71) durch eine Erweiterung (73) des Gehäuses (7) gebildet wird, die sich von einer Seitenwand (74) des ersten Gehäuses (7) aus erstreckt.

5. Shunt-Regulierungseinrichtung (1) nach Anspruch 4, wobei das Schlauchquetschventil (2) eine Drahtschlinge (23) umfasst, die in der Durchgangsöffnung (71) oder um dieselbe angeordnet ist.

6. Shunt-Regulierungseinrichtung (1) nach einem der vorhergehenden Ansprüche, wobei die elektrischen Antriebsmittel (3) ein Elektromotor sind.

7. Shunt-System (100), das eine Shunt-Regulierungseinrichtung (1) nach Anspruch 1 und eine zweite Steuereinheit (11) zur Anordnung außerhalb des Körpers (200) umfasst, wobei die zweite Steuereinheit (11) elektrisch mit einer ersten Drahlos-Sendeeinheit (10) verbunden ist, die dafür konfiguriert ist, von außerhalb des Körpers (200) an die erste Drahtlosempfangseinheit (6) ein Drahtlossignal zum Ferneinstellen des Schlauchquetschventils (2) zu senden.

8. Shunt-System (100) nach Anspruch 7, das ferner einen implantierbaren flexiblen Shunt-Schlauch (102) zum Befördern einer Körperflüssigkeit, mit einem ersten offenen Ende (103) und einem zweiten offenen Ende (104), umfasst.

9. Shunt-System (100) nach Anspruch 8, wobei der flexible Shunt-Schlauch (102) derart in der Durchgangsöffnung (71) des Gehäuses (7) angeordnet ist, dass das Schlauchquetschventil (2) zwischen dem ersten Ende (103) und dem zweiten Ende (104) des Shunt-Schlauchs (102) angeordnet ist.

10. Shunt-System (100) nach einem der Ansprüche 7 oder 9, wobei die zweite Steuereinheit (11) und die erste Drahlos-Sendeeinheit (10) in einer Fernsteuerung (12) angeordnet sind.

11. Shunt-System (100) nach einem der Ansprüche 7 bis 10, das ferner eine implantierbare Pumpe (101) umfasst, die auf ihrer Einlassseite (106) mit einer Einlasskanüle (105) zur Implantation in einer Herzkammer (202, 203) verbunden ist und auf ihrer Auslassseite (107) mit dem ersten offenen Ende (103) des flexiblen Shunt-Schlauchs (102) verbunden ist, wobei das zweite offene Ende (104) des Shunt-Schlauchs (102) in einem Blutgefäß (204, 205) implantierbar ist, das von der Herzkammer (202, 203) ausgeht.

12. Implantierbares Shunt-System (100) nach Anspruch 11, wobei die Pumpe (101) über ein Verbindungskabel (108) von außerhalb des Körpers (200), vorzugsweise über eine zweite Batterie (109), mit Elektroenergie versorgt werden kann.

13. Implantierbares Shunt-System (100) nach Anspruch 12, wobei die Pumpe (101) über das Verbindungskabel (108) elektrisch mit einer Pumpensteuereinheit (115) verbunden ist, die außerhalb des Körpers (200) angeordnet ist.

## Revendications

1. Dispositif de régulation de dérivation (1) destiné à l'implantation dans un corps (200), comprenant
a) un robinet à manchon à tube à entraînement électrique (2),
b) un moyen d'entraînement électrique (3) pour entraîner le robinet à manchon à tube (2) électriquement
c) au moins une première batterie (4) connectée électriquement au moyen d'entraînement électrique (3) pour alimenter le d'entraînement électrique (3) en énergie électrique,
d) une première unité de commande (5) connectée électriquement au moyen d'entraînement électrique (3) et configurée pour commander l'alimentation de l'énergie électrique au moyen d'entraînement électrique (3),
e) une première unité de réception sans fil (06) connectée électriquement à la première unité de commande (5) et étant configurée pour recevoir un signal sans fil externe, et
f) un premier logement fermé (7), une partie du premier logement (7) comprenant ou formant au moins une partie d'une ouverture de passage (71) pour recevoir un tube de dérivation flexible (102) ou un vaisseau du corps,
dans lequel
- au moins le moyen d'entraînement électrique (3) pour entraîner le robinet à manchon à tube (2) électriquement est agencé dans le premier logement fermé (7),
- l'au moins une batterie (4) et la première unité de commande (5) sont agencées, conjointement avec le moyen d'entraînement électrique (3), dans le premier logement (7), ou sont agencées conjointement dans un deuxième logement,
- le robinet à manchon à tube (2) est configuré de manière à ce que le robinet à manchon (2) soit en mesure de pincer un tube de dérivation flexible (102) ou un vaisseau du corps lorsqu'agencé dans l'ouverture de passage (71), et
- le robinet à manchon à tube (2) peut être ajusté depuis l'extérieur du corps (200) par le biais du signal sans fil externe.

2. Dispositif de régulation de dérivation (1) selon la revendication 1, dans lequel au moins une partie du robinet à manchon à tube (2), le moyen d'entraînement électrique (3), l'au moins une batterie (4) et la première unité de commande (5) sont agencés ensemble dans le premier logement (7).

3. Dispositif de régulation de dérivation (1) selon la revendication 1 ou 2, dans lequel une partie de base (72) du logement (7) comprend l'ouverture de passage (71).

4. Dispositif de régulation de dérivation (1) selon la revendication 1 ou 2, dans lequel l'ouverture de passage (71) est formée par une extension (73) du logement (7), s'étendant d'une paroi latérale (74) du premier logement (7).

5. Dispositif de régulation de dérivation (1) selon la revendication 4, dans lequel le robinet à manchon à tube (2) comprend une élingue à câble (23) agencée dans ou autour de l'ouverture de passage (71).

6. Dispositif de régulation de dérivation (1) selon l'une des revendications précédentes, dans lequel le moyen d'entraînement électrique (3) est un moteur électrique.

7. Système de dérivation (100), comprenant un dispositif de régulation de dérivation (1) selon la revendication 1 et une deuxième unité de commande (11) destinée à être agencée à l'extérieur du corps (200), la deuxième unité de commande (11) étant connectée électriquement à une première unité de transmission sans fil (10) configurée pour transmettre un signal sans fil à la première unité de réception sans fil (6) depuis l'extérieur du corps (200) pour ajuster le robinet à manchon à tube (2) à distance.

8. Système de dérivation (100) selon la revendication 7, comprenant en outre un tube de dérivation flexible implantable (102) pour transporter du fluide corporel, avec une première extrémité ouverte (103) et une deuxième extrémité ouverte (104).

9. Système de dérivation (100) selon la revendication 8, dans lequel le tube de dérivation flexible (102) est agencé dans l'ouverture de passage (71) du logement (7) de telle façon que le robinet à manchon à tube (2) soit agencé entre la première extrémité (103) et la deuxième extrémité (104) du tube de dérivation (102).

10. Système de dérivation (100) selon l'une des revendications 7 ou 9, dans lequel la deuxième unité de commande (11) et la première unité de transmission sans fil (10) sont agencées dans une commande à distance (12).

11. Système de dérivation (100) selon l'une des revendications 7 à 10, comprenant en outre une pompe implantable (101) qui, sur son côté entrée (106), est connectée à une canule d'entrée (105) pour l'implantation dans un ventricule cardiaque (202, 203) et sur son côté sortie (107), est connectée à la première extrémité ouverte (103) du tube de dérivation flexible (102), la deuxième extrémité ouverte (104) du tube de dérivation (102) étant implantable dans un vaisseau sanguin (204, 205) partant du ventricule cardiaque (202, 203).

12. Système de dérivation implantable (100) selon la revendication 11, dans lequel la pompe (101) peut être alimentée en énergie électrique depuis l'extérieur du corps (200) par un câble de connexion (108), de préférence par le biais d'une seconde batterie (109).

13. Système de dérivation implantable (100) selon la revendication 12, dans lequel la pompe (4) est connectée électriquement à une unité de commande de pompe (115) agencée à l'extérieur du corps (200) par le biais du câble de connexion (108).
